Europäisches Patentamt

European Patent Office

Office européen des brevets

(11) Publication number: **0 181 050**
**A2**

(12) **EUROPEAN PATENT APPLICATION**

(21) Application number: **85302644.1**

(22) Date of filing: **16.04.85**

(51) Int. Cl.⁴: **A 61 K 31/70**
**C 07 H 19/056**

(30) Priority: **29.10.84 US 666252**

(43) Date of publication of application:
**14.05.86 Bulletin 86/20**

(84) Designated Contracting States:
**AT CH DE FR GB IT LI NL SE**

(71) Applicant: **VIRATEK, INC.**
**3300 Hyland Avenue**
**Costa Mesa California 92626(US)**

(72) Inventor: **Smith, Robert Angus**
**221, 17 th Street**
**Santa Monica California 90402(US)**

(72) Inventor: **Fernandez, Humberto**
**3432, Eboe Street**
**Irvine California 92714(US)**

(74) Representative: **Goldin, Douglas Michael et al,**
**J.A. KEMP & CO. 14, South Square Gray's Inn**
**London WC1R 5EU(GB)**

(54) **1-b-d-ribofuranosyl-1,2,4-triazole-3-carboxamide for use in medical treatment of humans.**

㊗ The compound 1– ß–D–ribofuranosyl–1, 2, 4–triazole–3– carboxamide is utilized alone or in combination with other pharmacologic agents in the medical treatment of viral diseases in humans. Specific viruses against which the use of the invention is contemplated include Respiratory Syncytial Viruses (RSV), Retroviruses (RNA tumor viruses), Arenaviruses and Bunyaviruses.

Croydon Printing Company Ltd.

EP 0 181 050 A2

-1-

## DESCRIPTION

"1-β-D-RIBOFURANOSYL-1,2,4-TRIAZOLE-
3-CARBOXAMIDE FOR USE IN MEDICAL
TREATMENT OF HUMANS"

### Related U.S. Patent Documents

This Application is related to U. S. Patent No. 4,211,771 issued July 8, 1980 and Reissue of U. S. Patent No. 3,798,209, Reissue No. 29,835 reissued November 14, 1978. Both of these disclosures are incorporated herein by reference.

### Background of the Invention

The ongoing search for pharmacologic agents which are effective against various disease states caused by the over three hundred different immunologic types of virus is an important one. Viral disease accounts for some 60% of all diseases in humans. Among those viral disease states for which cures have been sought are:

(a) bronchiolitis, bronchitis, bronchopneumonia, tracheobronchitis, croup and febrile respiratory disease associated with Respiratory Syncytial Virus (RSV);

-2-

(b) Lassa Fever associated with hemorrhage, fever, pharyngitis, enteritis, myocarditis, pneumonitis, and, in fatal cases, circulatory collapse;

(c) Korean Hemorrhagic Fever associated with Arenaviruses and its clinical manifestations including severe toxemia, acute febrile illness, widespread capillary damage, hemorrhagic phenomena, severe central nervous system symptomatology and renal insufficiency (nephrosonephritis);

(d) Neoplasms, tumors, infections, and conditions such as Acquired Immune Deficiency Syndrome (AIDS) which is characterized by the development of Kaposi's Sarcoma and various infections; leukemias and lymphomas, associated with Retroviruses (RNA Tumor Viruses) and Adenoviruses;

(e) Viral infections in immunocompromised patients.

As a result of the significant morbidity and mortality of these viral diseases and the fact that efforts to develop an effective vaccine have thus far been unsuccessful, it is apparent that a chemotherapeutic agent effective against these disease states is needed. The search for effective antiviral pharmacotherapeutic agents is further complicated by the significant development of drug resistance by the various viruses to antiviral agents other than 1- ß-D-ribofuranosyl-1, 2, 4-triazole-3-carboxamide.

## Brief Summary of the Invention

The present invention relates to the use of the compound 1- ß-D-ribofuranosyl-1, 2, 4-triazole-3-carboxamide in the treatment of disease states caused by various viruses in humans. 1- ß-D-ribofuranosyl-1, 2, 4-triazole-3-carboxamide has been shown to exhibit broad spectrum antiviral activity both in vitro and in vivo. The compound may be administered either by injection, orally, topically, ophthalmically or via sprays or aerosol into the respiratory tract. Furthermore, the compound may be administered alone or in combination with other pharmacologic agents depending upon the disease state being treated.

Detailed Description of the Invention

United States Patent No. 3,798,209, which reissued November 14, 1978 as Re 29,835, disclosed the compound 1- ß-Dribofuranosyl-1, 2, 4-triazole-3-carboxamide as an antiviral agent exhibiting broad spectrum antiviral activity both in vitro and in vivo. Medical use of the compound in specific viral disease states was disclosed in United States Patent No. 4,211,771.

Below is a detailed description of the use of the compound 1- ß-D-ribofuranosyl-1, 2, 4-triazole-3-carboxamide alone or in combination with other pharmacologic agents in the treatment of various viral diseases. For the purposes of further illustrating the invention, 1- ß-D-ribofuranosyl-1,2,4triazole-3-carboxamide will be referred to interchangeably as (a) the compound, (b) Ribavirin (non-proprietary name adopted by the United States Adopted Names Council), or (c) under its chemical name above.

Respiratory Syncytial Virus (RSV) was first isolated in 1956 and was subsequently found to be the major lower respiratory tract pathogen of infancy and early childhood throughout the world. (Chanock, R.M., et al., Acute Respiratory Disease in Infancy and Childhood: Present Understanding and Prospects for Prevention. Pediatrics, Vol. 36, pages 21-39 (1965))

Of particular concern, but not limited only to, are infections in immunocompromised patients. For example, infants with Severe Combined Immunodeficiency Syndrome (SCID) are subject to overwhelming or prolonged infections with a number of viruses including RSV, influenza, parainfluenza and other RNA viruses. (Jarvis, W.R., et al., Significance of Viral Infections in Severe Combined Immunodeficiency Disease, Pediatr Infect Dis, Volume 2, pages 187-192 (1983))

As a result of the potential severity of symptomatology, occurrence and recurrence of these viral infections, the importance of finding a specific chemotherapeutic agent for the treatment of patients so infected is apparent. This concern is even further highlighted by the fact that efforts to develop an effective vaccine have thus far been unsuccessful. (Taber, L.H., et al., Ribavarin Aerosol Treatment of Bronchiolitis Associated with Respiratory Syncytial Virus Infection in Infants. Pediatrics, Volume 72, No. 5, pages 613-618 (November, 1983)).

The importance of finding an effective antiviral agent which is not subject to the development of drug resistance cannot be overemphasized in immunocompromised patients. Patients with compromised immune systems or with congenital heart and/or lung disease may require frequent and prolonged drug therapies thereby making conditions ripe for the development of drug resistance in susceptible agents. The development of resistant viral strains in the treatment of various viral diseases with, for example, Acyclovir, is a continuing problem. (See, Whittington W.L., et al., Acyclovir Therapy For Genital Herpes: Enthusiasm and Caution in Equal Doses, JAMA, Vol. 251 No. 16, April 27, 1984.) Drug resistance has not been found to occur with Ribavirin.

Among those other disease states for which a chemotherapeutic cure is sought are Lassa Fever and Korean Hemorrhagic Fever (KHF). Arenaviruses produce highly lethal hemorrhagic fevers, although milder and subclinical infections quite commonly occur also. While showing fewer hemorrhagic manifestations than, for example, Argentinian-Bolivian Hemorrhagic Fever, Lassa Fever causes severe pharyngitis, enteritis, myocarditis, and pneumonitis, and in fatal cases, circulatory collapse. (Fenner, F.J., et al., Medical Virology Second Edition, pages 416-417 (1976).) The etiologic agent in Lassa Fever is an Arenavirus endemic to regions of West Africa. The overall mortality rate of hospitalized cases reaches 15 to 20%.

Korean Hemorrhagic Fever also known as Hemorrhagic Fever with Renal Syndrome, Far Eastern Hemorrhagic Fever, endemic or epidemic nephrosonephritis, Manchurian Epidemic Hemorrhagic Fever, Songo Fever and Churilov's Disease, suggests an Arenavirus or Bunyavirus etiology. The hemorrhagic fever associated with KHF is more severe than that seen in Lassa Fever. Additionally, KHF is associated with a renal syndrome known as hemorrhagic nephrosonephritis. KHF is endemic to areas of Russia, China and Korea. Hemorrhagic Fever with Renal Syndrome (HFRS) is a debilitating and occasionally fatal disease of humans. As a result of the significant morbidity and mortality associated with these disease states, an effective chemotherapeutic antiviral agent is sought.

RNA viruses are known to cause malignant neoplasms in a wide range of species (Robinson, H.L., Rev. Infect. Dis. Volume 4, pages 1015-1025 (1982)). One such RNA virus named human T-cell Leukemia Virus (HTLV) has been associated with malignant proliferation of human T-lymphocytes. Identification of HTLV-like particles in patients with Acquired Immune Deficiency Syndrome (AIDS) also implicates this virus in the etiology of AIDS, which is characterized by immunodeficiency and the development of Kaposi's sarcoma. Furthermore, these patients are susceptible to various infections. (Essex, M., et al., Science, Volume 220, pages 859-871 (1983)).

Retrovirus-like particles containing RNA-directed DNA polymerase (reverse transcriptase) have also been isolated from human prostatic cells (Arya, S.K., et al., Oncology, Volume 37, pages 129-135 (1980)). Moreover, RNA-dependent polymerase activity has been found in human granulocytic sarcoma, human primary melanoma and human osteosarcoma tissue. (Chandra, P., Proc. Fed. Eur. Biochem. Soc., Volume 61, pages 215-218 (1980)). These studies suggest RNA oncogenic viruses as potential causative agents in certain types of human cancers.

The feature that unites RNA tumor viruses (retroviruses) and distinguishes them from all other animal viruses is the transcription of their single-stranded RNA into double stranded DNA and the presence of reverse transcriptase, an RNA-dependent DNA polymerase that is found in all RNA oncogenic viruses. Mutant RNA viruses lacking reverse transcriptase lose their ability to initiate infection and cell transformation. The presence of reverse transcriptase in all oncornaviruses strongly suggests its role in the neoplastic transformation of such viruses.

The compound can be administered to the human patient by injection, orally, topically, opthalmically, or via the respiratory tract in aerosols or drops. For systemic use, the compound would be given in an amount such that the total daily dose of the compound is from about 50 milligrams to about 2500 milligrams. The normal preferred range is from about 8.5 to 50 milligrams per kilogram of body weight per day. Depending on the mode of administration, the compound can be formulated with appropriate diluents to form solutions, suspensions, tablets, capsules, or syrups. For topical and ophthalmic use, the compound can be formulated with the appropriate diluents and carriers to form ointments, creams, foams, and solutions having from about 0.01% to about 15% by weight, preferably from about 1% to 10% by weight of the

-7-

compound. In any event, the actual amount should be sufficient to provide a chemotherapeutically effective amount of the compound to the patient, all of which will be readily within the ability of those skilled in the art to determine given the disclosure herein.

For a description of various illustrative dosage forms of the compound, reference should be made to the disclosure as set forth in United States Patent No. 4,211,771, the disclosure of which is incorporated herein by reference.

Representative examples of the use of the present invention in the treatment of various viral disease states in humans is discussed below. However, the examples are not in any sense limiting as a person skilled in the art may use the present invention in other ways in the treatment of viral diseases. The invention is limited only by the scope of the appended claims.

EXAMPLE 1

Respiratory Syncytial Virus (RSV)

Respiratory Syncytial Virus (RSV) is an RNA virus which is enveloped by a lipoprotein coat. (BERTHIAUME, L., et al., Comparative Structure, Morphogenesis and Biological Characteristics of the Respiratory Syncytial (RS) Virus and the Pneumonia Virus of Mice, ARCH GESAMTE VIRUSFORSCH, Vol. 45, pages 39-51 (1974)) The virus matures at the limiting membrane of the affected cell. (Kalica, A.R., et al., Electron Microscopic Studies of Respiratory Syncytial Temperature-Sensitive Mutants. ARCH GESAMTE VIRUSFORSCH, Vol. 41, pages 248-258 (1973)) Since it characteristically induces formation of large syncytial masses in infected tissue cultures it was termed Respiratory Syncytial Virus.

Thirty-three infants aged 6-days to one year with clinical signs of bronchiolitis and subsequently confirmed to have RSV infection were randomly consigned to have aqueous solution of ribavirin 20 mg/ml in the nebulizer chamber or water by aerosol for approximately 18 hours per day for total dosing periods of 61-91 hours over 3-5 days. At daily physical examinations the following features of the illness were noted on a 0-3 scale; nasal signs, inflammation of tonsils, cough, apnea, cyanosis, crepitations, wheezing, hyperinflation, chest wall retraction, tracheobronchitis, pneumonia, anorexia, malaise, stridor, clinical signs of toxicity and temperature. Nasal washes were conducted daily and the titer of virus shed was noted.

When compared with placebo treatment, ribavirin treatment was associated with a markedly greater improvement in the following indices: cough, cyanosis, crepitations, wheezing, chest retraction pneumonia, malaise and viral shedding at the end of therapy. Severity of illness scores derived from the clinical findings showed significantly lower values for the ribavirin than for the placebo groups by days 3 and 4 (1.6 c.f. 2.5, $p = 0.016$ on day 3; 1 c.f. 2.4, $p = 0.01$ on day 4). Virus shedding titers were significantly lower in the ribavirin than the placebo groups by the end of therapy (6.9 c.f. 4443.2 log $TCID_{50}$/ml ($p < 0.01$). The number of patients improved at the end of treatment was significantly higher in the ribavirin than in the placebo group ($p = 0.02$).

Sixteen young healthy adults received 1 ml of an RSV challenge pool intranasally on day 1 and were then isolated and randomly consigned in double-blind manner to treat with ribavirin or water. These were administered by small particle aerosol starting on day 3 post inoculation. Treatment was administered for 12 hours/day for 3 days. The concentration of ribavirin in the nebulizer chamber was 20 mg/ml. All subjects were assessed daily by the same physician and relevant symptoms were graded for severity on a 0-3 scale. Nasal wash specimens for viral titers were taken and pulmonary function tests conducted on all subjects prior to administration of the virus and twice daily thereafter. Maximal expiratory function tests before and after carbachol administration were performed prior to virus administration, at the end of aerosol administration and again 2 months later. Seven of the 8 placebo treated and 6 of 8 ribavirin treated subjects became infected.

Ribavirin had little apparent effect on minor upper respiratory tract signs but tracheobronchitis, systemic illness and fever occurred significantly less often ($p < 0.01$) in the ribavirin treated group. Virus shedding was diminished in the ribavirin treated group and the proportion of infected volunteers still shedding virus on days 6-8 was also lower in the drug treated subjects ($p = 0.046$). Pulmonary function testing did not yield evidence of any significant difference between the groups.

An infant with Severe Combined Immunodeficiency Syndrome (SCIDS) secondary to adenosine deaminase deficiency had pneumonitis and combined infection with Respiratory Syncytial Virus (RSV) and parainfluenza virus type 3 (PIV3). Four separate courses of Ribavirin were delivered by small-particle aerosol. The drug was administered by a small-particle nebulizer into the patient's oxygen tent daily during a 20 hour period. The patient received two 5-day (days 9-14 and 22-27) and two 10-day (days 33-43 and 59-69) courses of treatment. The reservoir concentration of the drug during the first two courses of treatment was 20 mg/ml and was increased to 25 mg/ml during the subsequent two longer courses. The 5-month-old infant's symptomatology upon admittance included mild respiratory distress, heightened pulse and respiratory rates, fever, wet cough, bilateral rales, a diffuse, fine maculopapular rash on her trunk and lower

-10-

extremities and an erythematous rash with satellite lesions. RSV and Parainfluenza Virus 3 (PIV3) were identified by rapid immunofluorescence of epithelial cells that were shed into the nasopharynx.

The patient demonstrated a slow but dramatic change throughout the ten weeks during which Ribavirin was intermittently administered. All areas of pulmonary assessment improved. The PIV3 disappeared from the respiratory secretions during the first course of Ribavirin and did not return. After the second 5-day course of Ribavirin, excretion of RSV became intermittent and ceased several days in the fourth course. Throughout the extended therapeutic protocol no significant change in Ribavirin sensitivity was observed.

EXAMPLE II

Lassa Fever

The overall mortality rate in hospitalized patients with Lassa Fever reaches 15 to 20%. Patients with Lassa Fever may be subdivided by two indicators or mortality risk; admission levels of AST (SGOT) and viremia. An admission serum AST $\geq$ 150 carries a risk of mortality of 50% while an admission viremia $\geq 10^4$ $TCID_{50}$/ml carries a mortality rate of about 70%.

Chemotherapeutic trials in Lassa Fever patients were initiated with a trial of oral ribavirin, followed by a trial of higher dose intravenous ribavirin in an attempt to improve survival from that observed with oral ribavirin.

Patients were randomly divided into two subgroups receiving either 15 mg/kg orally of Ribavirin in three divided doses per day (approximately 1000 mg/kg/day) over a 10-day period; or one unit of Lassa immune plasma with an IFA titer of $\geq$ 1:128.

In the second trial, patients with admission AST values $\geq$ 150 i.u. were randomly divided into two subgroups. One group received two grams of intraveneous Ribavirin followed by one gram every six hours for four days. The dose was then reduced to 0.5 gm every eight hours for six more days. The second group received the same dose of Ribavirin and one unit of plasma given with the loading dose of Ribavirin.

Patients treated with oral or i.v. Ribavirin who had admission AST values < 150 had a mortality of 20% compared to 51% in patients who were untreated or who received one unit of plasma and compared to 38% in patients who received two units of plasma. In patients treated with Ribavirin (oral or i.v.) who had admission viremias of 10 3.6 TCID/ml, mortality was 31% compared to 71% in patients who were either untreated or who received one unit of immune plasma. Both comparisons represent a reduction in mortality rate of more than 50%.


EXAMPLE III

Korean Hemorrhagic Fever (KHF)

A group of closely related viruses that cause hemorrhagic fever with associated renal syndrome in hundreds of thousands of human cases each year has received increased attention with the recognition of the serological relationship between Korean Hemorrhagic Fever and a number of similar diseases worldwide. In vitro testing has shown sensitivity of the virus to Ribavirin. In vivo studies utilizing the striped field mouse Apodemus agarius have also shown sensitivity to Ribavirin. Treatment of infected animals with Ribavirin at 25 mg/kg/day over nine days resulted in 3 of 4 animals with antigen negative lungs, and reduced KHF antigen in the fourth.

In another study the Hantaan virus (HV) suckling mouse model was used to evaluate the efficiency of Ribavirin treatment starting at 6, 10 or 14 days post infection with varying doses of drug. Animals infected

with 10 $LD_{50}$'s of HV (strain 76/118) showed weight loss beginning on day 10 and untreated animals developed paralysis of both hind limbs on days 15-18 followed by death. Treatment with 50 mg/kg begun on day 10 following onset of early clinical signs and demonstrable virus in serum and organs, saved 11/20 animals compared to 0/70 controls. Treated animals did not develop symptoms and by day 22 survivors resumed normal weight gain. The successful therapeutic intervention following onset of clinical and virological findings suggests that similar therapy in humans might be effective.

0181050

- 13 -

CLAIMS

1. The use of the compound 1-β-D-ribofuranosyl-1,2,4-triazole-3-carboxamide for the manufacture of a pharmaceutical composition for the medical treatment of viral diseases, in humans, caused by Respiratory Syncytial viruses, Retroviruses, Arboviruses, Arenaviruses or Bunyaviruses.

2. The use according to claim 1, characterised in that said viral disease occurs in immunocompromised patients.

3. The use according to claim 1, characterised in that said viral disease occurs in patients with congenital heart and/or lung disease.

4. The use according to claim 1, characterised in that said viral disease caused by Respiratory Syncytial virus occurs in patients with Severe Combined Immune Deficiency Syndrome.

5. The use according to any one of claims 1 to 4, characterised in that said viruses are capable of developing resistance to antiviral agents.

6. The use according to any one of claims 1 to 5, characterised in that said compound is present in the pharmaceutical composition to be administered to a human patient in an amount from about 0.01 % to 50 % by weight based on the total weight of the composition.

7. The use according to any one of claims 1 to 6, characterised in that the composition is in a form suitable for oral administration, topical administration, administration by injection, administration via the respiratory tract or ophthalmical administration.

8. The use according to any one of claims 1 to 7, characterised in that the compound is used for the manufacture of an aerosol preparation containing said compound in a total daily dose of approximately 40 to 60 mg/kg for the medical treatment of viral diseases caused by Respiratory Syncytial viruses in human patients with Severe Combined Immune Deficiency Syndrome.

9. The use according to any one of claims 1 to 3 or 5 to 7, characterised in that the compound is used for the manufacture of an oral preparation containing said compound in a total daily dose of approximately 10 to 100 mg/kg for the medical treatment of viral diseases caused by Arenaviruses in human patients.

10. The use according to any one of claims 1 to 3 or 5 to 7, characterised in that the compound is used for the manufacture of a composition to be administered intravenously in a total daily dose of approximately 1g to 4g for the medical treatment of viral diseases caused by Arenaviruses in human patients.